# EUROPEAN PATENT SPECIFICATION

(11) **EP 0 334 165 B1**
(45) Date of publication and mention of the grant of the patent: **27.12.1995**
(21) Application number: 89104494.3
(22) Date of filing: 14.03.1989
(51) Int. Cl.: C12P 21/00, A61K 39/395, G01N 33/68, G01N 33/577, C07K 1/00

(54) **Monoclonal antibodies**
Monoklonale Antikörper
Anticorps monoclonaux

(30) Priority: 17.03.1988 GB 8806339
(43) Date of publication of application: 27.09.1989
(73) Proprietor: F. HOFFMANN-LA ROCHE AG, 4002 Basel (CH)
(72) Inventor: Brockhaus, Manfred, Dr., CH-4126 Bettingen (CH); Lötscher, Hansruedi, Dr., CH-4313 Möhlin (CH)
(74) Representative: Lederer, Franz, Dr.

(56) References cited:
- THE JOURNAL OF IMMUNOLOGY, vol. 138, no. 6, 15th March 1987, pages 1786-1790; P. SCHEURICH et al.: "Immunoregulatory activity of recombinant human tumor necrosis factor (TNF)-alpha: Induction of TNF receptors on human T cells and TNF-alpha-mediated enhancement of T cell responses"
- IMMUNOLOGY LETTERS, vol. 12, 1986, pages 217-224; S. ISREAL et al.: "Binding of human tnf-alpha to high-affinity cell surface receptors: effect of ifn"
- THE JOURNAL OF IMMUNOLOGY, vol. 139, no. 4, 15th August 1987, pp. 1161-1167; H. HOLTMANN et al.: "Down regulation of the receptors for tumor necrosis factor by interleukin 1 and 4beta-phorbol-12-myristate-13-acetate"

## Description

Tumour Necrosis Factor (TNF) is a protein released by activated macrophages in response to external stimuli like, for example, endotoxin. It was originally found in the serum of mice treated with Bacillus Calmette-Guerin and bacterial endotoxin. Its characteristic effect in vivo is the production of necrosis in experimental animal tumours [Carswell, E.A. et al. (1975), Proc. Natl. Acad. Sci. USA, 72, 3666; Matthews, N. & Watkins, J.F. (1978), Br. J. Cancer, 38, 302; Matthews, N. (1978), Br. J. Cancer, 38, 310]. It has diverse biological effects in other experimental systems including killing of tumour cells in vitro [Old, L.J. (1985) Science 230, 630], inhibition of the activity of lipoprotein lipase [Beutler, B. & Cerami, A. (1987), New Engl. J. Med. 316, 379], mediation of some of the lethal effects of endotoxin in animals [Beutler, B. et al. (1985), Science, 229, 869], stimulation of granulocytes and fibroblasts [Old, L.J. (1985), Science, 230, 630; Beutler, B. & Cerami, A. (1987), New Engl. J. Med. 316, 379; Vilcek, J. et al. (1986), J. Exp. Med. 163, 632), damage to endothelial cells [Sato, N. et al. (1986), J. Natl. Cancer Inst. 76, 1113], bone resorption [Bertolini, D.R. et al. (1986), Nature 319, 516), antiviral activity [Mestan, J. et al. (1986), Nature 323, 816; Wong, G.M.W. & Goeddel, D.V. (1986), Nature 323, 819] and cytotoxic effects against malaria parasites [Taverne, J. et al. (1984), Clin. Exp. Immunol. 57, 293]. Some of these effects are probably mediated via the induction of other secreted factors. The effects of TNF are synergised in part by interferon-γ, interleukin-1 and the bacterial lipopolysaccharides (LPS).

Macrophage or monocyte-produced TNF is also referred to as TNF-α or cachectin to distinguish it from TNF-β, a closely related lymphocyte-product which is also called lymphotoxin [Pennica et al. (1984), Nature 312, 724]. Both human TNF-α and TNF-β have been cloned and expressed in E. coli making large quantities of the corresponding proteins available [Pennica, D. et al., see above; Shirai, T. et al. (1985), Nature 313, 803; Wang, A.M. et al. (1985), Science 228, 149; Gray et al. (1984), Nature 312, 712]. Structural comparison of TNF-α and TNF-β at the protein sequence level shows about 30% homology [Pennica et al., see above].

According to Pennica et al. (1984) and Gray et al. (1984) both TNFs have indistinguishable biological activities and bind to the same cell surface receptor (TNF-R) [Aggarwal, B.B. et al. (1985) Nature 318, 665] or receptor complex which is believed to be responsible for the transduction of the TNF signal to the intracellular compartment. Cells have in the range of 5000 binding sites per cell with a high affinity for recombinant TNT (K_{D}: 0.1 to 1 nM) [Tsujimoto, M. et al. (1985), Proc. Natl. Acad. Sci. U.S.A. 82, 7626 and Baglioni, C. et al. (1985), J. Biol. Chem. 260, 12295 and others]. After binding to the cell surface receptor TNF becomes quickly internalised by the cell [Tsujimoto, M. et al., see above and Shalaby, M.R. et al. (1987), J. Leukocyte Biol. 41, 196]. A TNF binding protein - or at least part of a putative TNF-receptor complex - has been identified by chemical crosslinking studies with ¹²⁵I labelled TNF. It has been described as a 70-80 kD molecule [Kull, F.C. et al. (1985), Proc. Natl. Acad. Sci. U.S.A., 82, 5756 and Tsujimoto, M. et al., see above]. Other proteins of higher molecular weight have also been described to crosslink to TNT [Creasey, A.A. et al. (1987), Proc. Natl. Acad. Sci. U.S.A. 84, 3293]. These proteins might be part of a putative receptor complex or the products of post-translational modifications of a single receptor protein.

TNF-α has been shown to be involved in a number of pathological conditions in man and mouse like septic shock from meningococcal bacteremia in humans [Waage, A. et al. (1987), Lancet, Feb. 14th, 355], parasitic diseases [Scuderi, P. et al. (1986), Lancet, Dec. 13th, 1364], development of cytotoxic T cells in mice [Ranges, G.E. et al. (1987), J. Exp. Med. 166, 991], development of glomerulonephritis in autoimmune mice [Jacob, C.O. and McDevitt, H.O. (1988) Nature 331, 356], graft versus host disease in mice [Piguet, P.-F. et al. (1987), J. Exp. Med. 166, 1280], development of cerebral malaria in mice [Grau, G. et al. (1987), Science 237, 1210] and Kawasaki syndrome [Donald, Y.M. et al. (1986), J. Exp. Med. 164, 1958]. A possible role in pathological conditions like the destruction of pancreatic beta-cells [Mandrup-Poulsen, T. et al. (1987), Acta endrocrinologica 115, Supplement 282, 40] which leads to insulin-dependent diabetes mellitus has also been attributed to TNF itself and its functioning as an activator of secretion of interleukin-1. TNF-α has been shown to be involved in endotoxin induced shock in rats (Tracey, K.J. et al (1986), Science 234, 470) and baboons (Tracey, K.J. et al. (1987), Nature, 330, 662-664) and in cachexia due to parasitic infections or neoplasia (reviewed by Beutler, B. and Cerami, A. (1987), New Engl. J. Medicine, 316, 379). The pathogenic effects of TNF-α and TNF-β are at least in part attributed to damage of the endothelial cell wall in the vascular system [Stolpen, A.H. et al. (1986), Am. J. P. 123, 16] and [Pober, J.S. (1987), J. Immunol. 138, 3319]. In addition the adhesiveness of neutrophils to the endothelium is greatly enhanced by TNF (Cavender, D. et al. (1987), J. Immunol. 139, 1855).

It is an object of the present invention to provide monoclonal antibodies against a human cell surface TNF binding protein, in the following called receptor for human Tumor Necrosis Factor (hTNF-R), biologically active fragments thereof, whereby fragments with intact antigen binding capability are preferred, hybridoma cell lines secreting said antibodies, processes for the preparation of said antibodies or fragments thereof, pharmaceutical compositions containing said antibodies or fragments thereof and to describe the use of such antibodies or fragments in the treatment or diagnosis of various diseases or in the purification of the hTNF-R.

Monoclonal antibodies of the present invention can be manufactured as follows:
For the preparation of the antigen human cells which carry the TNF-R like granulocytes or cells from placental tissue or cells from cell lines like adenocarcinomas, for example, ZR-75, MCF-7, HEp-2, lymphomas or Epstein Barr Virus transformed B-cells are cultured under conditions generally known in cell culture technology. Cells are harvested and TNF-Rs are solubilized in the presence of detergents like Triton or the like where Triton X 114 is particularly preferred and protease inhibitors like, for example, PMSF. Further purification of receptors can be achieved by various chromatographic procedures like, for example, ion exchange chromatography, gel filtration or high performance liquid chromatography (HPLC), with affinity chromatography on the basis of the receptor ligand fixed to a solid support being preferred in the purification procedure of the present invention.

By injection of such a purified antigen into a mouse, rabbit, rat, sheep or the like polyclonal antibodies can be obtained from the serum or monoclonal antibodies can be prepared by recovering antibody producing cells from such an immunized animal and immortilizing said cells obtained in conventional fashion like fusion with myeloma cells with PAI mouse myeloma cells being particularly preferred. Supernatants of cultures of such hybridomas are screened for monoclonal antibodies by conventional procedures like radioimmuno- or enzymimmuno- or dotimmunobinding assays or a downmodulation assay as described in Example 5 or by an capture assay as described in detail in Example 4. Radioiodinated TNF-α used in this assay can be prepared for example by the chloramin-T-, the lactoperoxidase- or the Bolton-Hunter method or in accordance with description of Example 4 hereinafter.

Monoclonal antibodies can be purified from hybridoma supernatants by conventional chromatographic procedures like, for example, by Ion-exchange-, affinity chromatography on protein A or anti-immunoglobulin/antibodies bound to a solid support, HPLC or the like.

For the production of large quantities of monoclonal antibodies in accordance with methods well-known in the art hybridomas secreting the desired antibody can be injected intraperitoneally into mice which have been pretreated with pristane for example before injection. Up to around 100 mg of a monoclonal antibody can be produced by such ascites tumors in one mice. Antibodies can be purified for example from ascites fluid produced by such tumors using methods as stated above.

Monoclonal antibodies can be characterized according to their subclass by known methods such as Ouchterlony immunodiffusion. In the present invention two monoclonal antibodies, namely the ones secreted by hybridoma cell lines htr-1c and htr-2a were of the IgM subtype, three monoclonal antibodies, namely the ones secreted by hybridoma cell lines htr-4a, htr-5a and htr-9b were of the IgG1 subclass and two monoclonal antibodies, namely the ones secreted by the hybridoma cell lines htr-6b and htr-7b were of the IgG2b subclass.

The monoclonal antibodies of the present invention are characterized by a "downmodulation" on human cells. The degree of this downmodulation is measured on specific cells, in particular on HL-60 and HEp-2 cells. The term "downmodulation" refers to the following process. If a monoclonal antibody of the present invention is bound to living cells carrying TNF-R under physiological conditions the cells loose their ability to bind TNF. This will be better understood on the basis of the detailed description of the experimental conditions given in Example 5 hereinafter in connection with the accompanying drawings.

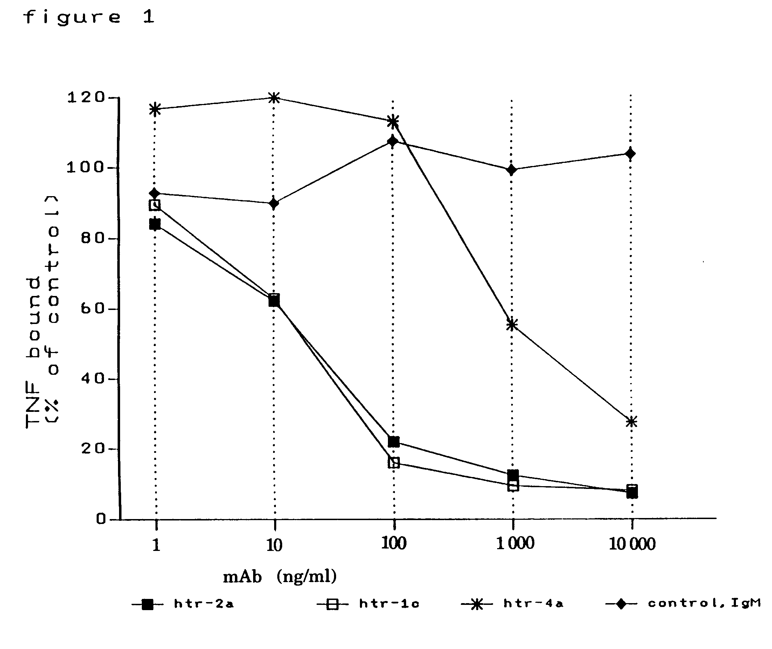

Figure 1 shows the downmodulation of TNF-binding to HL 60 [ATCC No. CCL 240] cells where "TNF bound" refers to the amount of ¹²⁵I-TNF as percent of radioactivity bound to cells not treated with TNF-R antibodies ("control"). " 1 ng ", for example, refers to the amount of monoclonal antibody (mAb) used with the type of antibody being indicated by different symbols below with the specific name of the antibody behind it. According to the procedure described in Example 5 human cell lines growing in suspension can be tested for downmodulation of TNF-binding. "EC₅₀" refers to the concentration of a monoclonal antibody of the present invention where 50% of TNF as per cent of control is still bound to the cells.

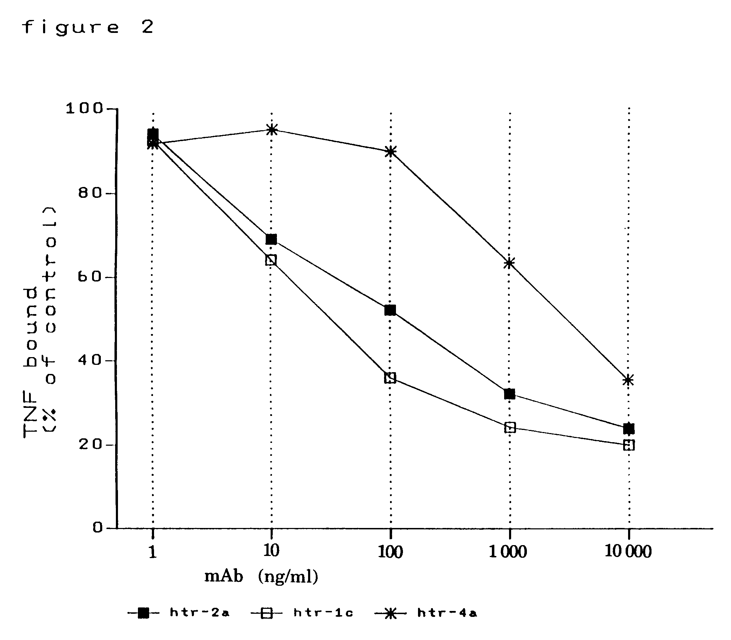

Figure 2 illustrates the same phenomenon for HEp-2 cells [ATCC No. CCL 23] as an example of adherent cells.

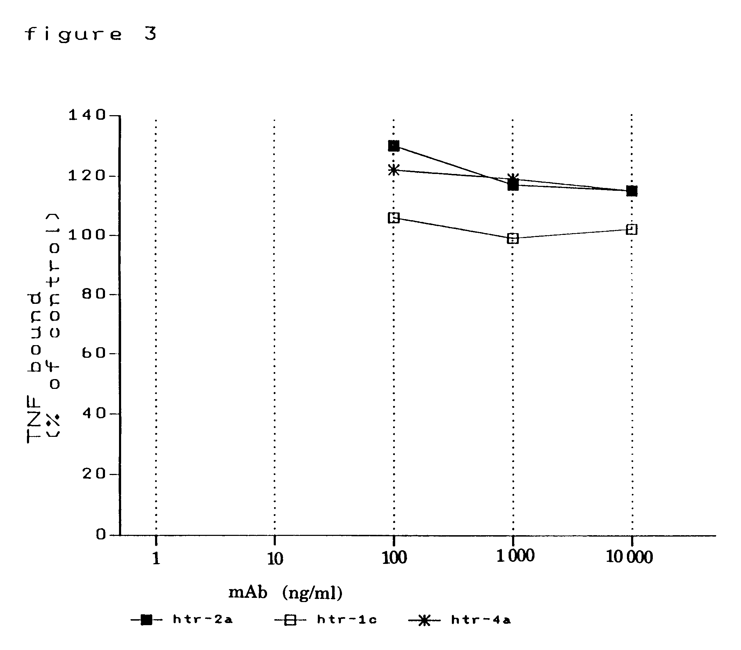

The monoclonal antibodies of the present invention do not downmodulate TNF-binding on murine cells shown for WEHI-164 cells [ATCC No. CRL 1751] in Figure 3.

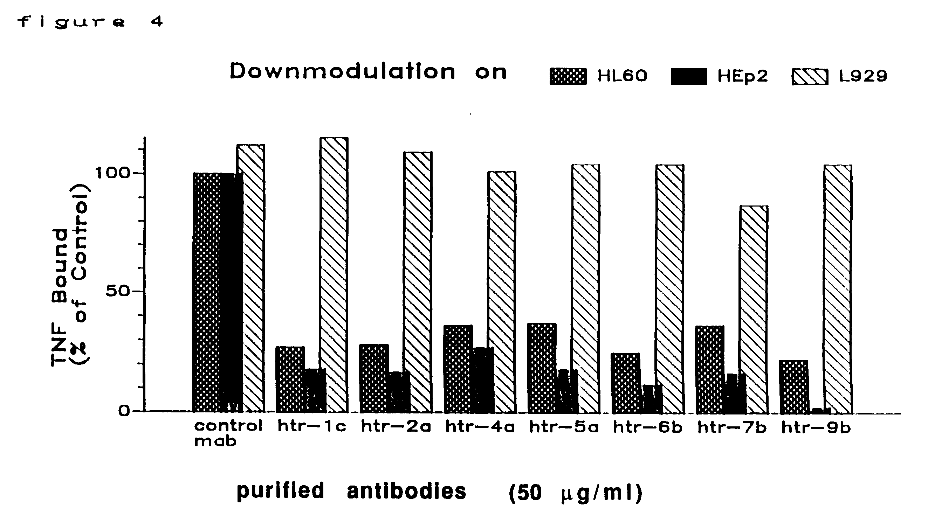

Figure 4 gives a direct comparison of the degree of downmodulation of specific examples of mAbs (htr-1c, htr-2a, htr-4a, htr-5a, htr-6b, htr-7b, htr-9b) of the present invention on HL-60 cells and HEp-2 cells with no down modulation on L-929 cells [ATCC No. CCL 1].

Downmodulation can be determined as stated above on all human cells. Preferably such downmodulation is determined on HL 60 cells with IgM antibodies of the present invention showing on such cells an EC₅₀ of about 30 ng/ml or with IgG antibodies of the present invention showing on such cells an EC₅₀ of about 2.5 »g/ml. In another preferred embodiment such downmodulation is determined on HEp-2 cells with IgM antibodies of the present invention showing on such cells an EC₅₀ in the range of about 50 ng/ml to about 170 ng/ml or with IgG antibodies of the present invention showing on such cells an EC₅₀ of about 5.5 »g/ml. Downmodulation is also described by Aggarwal, B.B. and Eessalu, T.E. [J. Biol. Chem. (1987), 262, 16450] and Holtmann, H. and Wallach, D. [J. Immunol. (1987) 139, 1161] as "downregulation" of TNF-Rs caused by the administration of certain phorbol esters which are known to act on the enzymes involved in intracellular signalling pathways.
htr-9b are specific examples of hybridoma cell lines useful in the present invention. These have been deposited at the European Collection of Animal Cell Cultures (ECACC) in Salisbury (Great Britain) on March 10th, 1988 under the terms and conditions of the Budapest Treaty with the following ECACC accession numbers: 88031001 for htr-1c, 88031002 for htr-2a, 88031003 for htr-4a, 88031004 for htr-5a, 88031005 for htr-6b, 88031006 for htr-7b and 88031007 for htr-9b.

It is, however, to be understood that the present invention is not restricted to the use of those specific hybridoma cell lines.

Is is well known in the art that monoclonal antibodies can be modified for various uses or fragments thereof can be generated which still show binding of the antigen. Such fragments can be generated, for example, by enzymatic digestion of antibodies with Papain, Pepsin or the like.

The monoclonal antibodies of the present invention can be used for the immunoaffinity purification of the TNF-R. Therefore such antibodies can be linked to solid supports by methods well-known in the art, for example by covalent binding to CNBr activated agarose, such as Sepharose, or the like.

Furthermore, the monoclonal antibodies of the present invention can be used as a diagnostic tool for the determination of TNF-Rs on the cell surface and of putative soluble forms of TNF-RS. TNF-R carrying cells can be localized in culture or on tissue samples. For such use antibodies are coupled, for example, to a fluorescent dye, a colour producing substance, like an enzyme [enzyme linked immunosorbent assay (ELISA)] or a radioactive substance [radioimmunoassay (RIA)] in accordance with methods well-known in the art.

Monoclonal antibodies of the present invention may be used in the treatment of various diseases with treatment of septic shock being particularly preferred. Said antibodies can be used if necessary in combination with other pharmaceutically active substances and/or with conventionally used pharmaceutically acceptable solid or liquid carrier materials. Dosage and dose rates may be choosen in analogy to dosage and dose rates of currently used antibodies in clinical treatment of various diseases. To diminish sensitization of the recipient against antibodies of the present invention chimeric antibodies of human/mouse origin generated according to known methods in the art may be used.

Having now generally described this invention, the same is illustrated by the following examples which are in no way to be interpreted as limiting.

### Example 1

### Purification of the Antigen (TNF-R)

HL-60 cells [ATCC No. CCL 240] were grown in a RPMI 1640 medium [GIBCO Catalogue No. 074-01800] containing 2 g/l NaHCO₃ and supplemented with 5% fetal calf serum in a 5% CO₂ atmosphere to a density of 1.5 x 10⁶ cells/ml. The cells (3 x 10¹⁰) were washed in PBS and lysed at 0°C by adding 100 ml PBS with 1% Triton X 114 and 1 mM PMSF to the pellet. The lysate was centrifuged at 0°C for 30 mins. at 20 000 g and the supernatant was diluted 1:10 with PBS. The diluted supernatant was applied at 4°C onto a column (flow rate: 0.2 ml/min.) containing 2 ml affigel 10 (Bio Rad Catalogue No. 153-6099) to which 20 mg recombinant human TNF-α [Pennica, D. et al. (1984) Nature 312, 724; Shirai, T. et al. (1985) Nature 313, 803; Wang, A.,M. et al. (1985) Science 228, 149] have been linked to according to the manufacturers recommendations. The column was washed at 4°C at a flow rate of 1 ml/min first with 20 ml PBS containing 0.1% Triton X 114 and second with 20 ml PBS alone. The receptor was eluted at 22°C at a flow rate of 2 ml/min from the column with 4 ml 100 mM glycin, pH 2.8, 0.1% decylmaltosid. The eluate was concentrated in a Centricon 30 tube [Amicon] to 10 »l.

### Example 2

### Immunisation

10 »l of the concentrated eluate were mixed with 20 »l complete Freund's adjuvans. 10 »l of the emulsion were injected according to a method as described by Holmdahl, R. et al. [(1985), J. Immunol. Methods 83, 379] into one rear foot pad of an anaesthesized Balb/c mouse at days 0, 7 and 12.

### Example 3

### Preparation of Hybridoma Cell Fusions

At day 14 the immunized mouse was sacrificed and the popliteal lymphnode was taken and minced and suspended in Iscove's medium (IMEM, GIBCO catalogue no. 074-2200) containing 2 g/l NaHCO₃ by repeated pipetting. Following a modification of the procedure of De St.Groth and Scheidegger [J. Immunol. Methods. (1980), 35, 1] 5x10⁷ lymphnode cells were fused with 5 x 10⁷ PAI mouse myeloma cells (J.W. Stocker et al., Research Disclosure, 217, May 1982, pp. 155-157) growing in logarithmic phase. The cells were mixed, pelleted by centrifugation and resuspended under gentle agitation in 2.0 ml of 50% (v/v) polyethylene glycol in IMEM at room temperature and diluted by adding slowly 10 ml IMEM over 10 min of gentle agitation. The cells were pelleted by centrifugation and resuspended in 200 ml complete medium [IMEM + 20% fetal bovine serum, glutamine (2.0 mM), and 2-mercaptoethanol (100 »M) containing 100 »M hypoxanthine, 0.4 »M aminopterine, and 16 »M thymidine (HAT)]. The suspension was dispersed over 10 96-well tissue culture plates and incubated for 11 days without change of medium at 37°C in an atmosphere of 5% CO₂ and 98% humidity.

### Example 4

### Screening for Hybridomas secreting Antibodies against hTNF-R

Hybridoma culture supernatants (100 »l) were transfered to ELISA plates (Nunc) coated overnight with conventional affinity purified rabbit anti-mouse Ig antibodies (10 »g/ml PBS at 4°C). The supernatants were replaced after 2 hrs by a solution of 1% BSA and 0.01% mouse Ig in PBS and incubated for 2 more hours. 3 x 10⁹ HL 60 cells in 50 ml RPMI 1640 medium supplemented with 5% fetal bovine serum and 0.1% sodium azide were incubated with 1 »g/ml TNF-α for 1 hr at 37°C, washed once with PBS and lysed, centrifuged and diluted like it has been described in example 1. The diluted lysate was transferred to the ELISA plates (50 »l/well), incubated for 2 hrs at 4°C and washed out with 4 washes of 0.1% Triton X 114 in PBS at 4°C. A monoclonal antibody against TNF which was generated as described in example 3 and selected by ELISA as described by Bringman, T.S. et al. [(1987), Hybridoma 6, 489] was labelled with ¹²⁵I and Iodo-Gen (Pierce Catalogue no. 28600) according to a procedure published by Fraker, P.J. and Speck, J.C. [(1978), Biochem. Biophys. Res. Commun. 80, 849]. The ¹²⁵I-labelled anti-TNF antibody (2000 Ci/mmol) diluted in PBS with 1% BSA to 10⁶ cpm/ml was added to the wells (100 »l) and incubated for 2 hrs at 22°C. The wells were washed extensively with PBS and autoradiographed over night on a Kodak Xomat XR-5 film with a Dupont Chronex screen. The hybridomas which were selected by a positive signal in this assay secrete antibodies which are either directed against the hTNF-R or TNF. The hTNF-R antibodies were identified by their inability to bind to TNF coated ELISA plates and by their biological activity described in example 5. The heavy chain isotype of the antibodies was determined by precipitation in Ouchterlony agarose plates with either goat anti-mouse IgG isotype or anti-mouse/IgM isotype specific sera (Nordic).

### Example 5

### Downmodulation of TNF-binding

5 x 10⁶ HL 60 cells (example 1) were incubated in complete RPMI 1640 medium together with affinity purified monoclonal antibodies as obtained by a process according to examples 1-4 or control antibody of unrelated specificity in a range of concentration between 1 ng/ml and 10 »g/ml. After 1 hr of incubation at 37°C the cells were pelleted by centrifugation and washed with 4.5 ml PBS at 0°C. They were resuspended in 1 ml complete RPMI 1640 medium (example 1) containing additional 0.1% sodium azide and ¹²⁵I-TNF (10⁶ cpm/ml). The ¹²⁵I-labelled TNF had been obtained from TNF, Iodo-Gen (Pierce Cat. No. 28600) according to the procedure described by Fraker and Speck (example 4). The specific radioactivity was 700 Ci/mmol. The cells were incubated for 2 hrs at 4°C, pelleted and washed 4 times with 4.5 ml PBS containing 1% BSA and 0.001% Triton X 100 (Fluka) at 0°C. The cell bound radioactivity was measured in a γ-scintillation counter. In an analogous experiment the cell bound radioactivity of cells which have not been treated with anti-TNF receptor antibodies before was determined to be 10 000 cpm/5x10⁶ cells. An analogous protocol was used for the assay of WEHI-164-, L929- and HEp-2-cells omitting the steps of centrifugation in the case of adherent cell lines. The results of such assays on HL60-, HEp-2- WEHI-164 and L929 cells are shown in figures 1-4.

### Example 6

### Generation of F_{ab}-fragments by papain treatment

To 0.5 ml of a solution of the monoclonal antibody "htr-9" (10 mg/ml) [prepared as described in examples 1-4] in 75 mM sodiumphosphate, 75 mM sodiumchloride and 2 mM EDTA pH 7.0, 0.1 ml papain (Boehringer Mannheim, Mannheim, BRD) was added as a freshly prepared solution containing 100 »g/ml enzyme in the same sodiumphosphate/sodiumchloride/EDTA buffer containing 60 mM cystein. After incubation of 1 hour at 37°C the reaction was stopped by the addition of 10 »l of a freshly prepared solution of iodoacetamide (1M) in water. The mixture was kept 30 minutes at room temperature in the dark and 60 »l of a 2M solution of Tris/HCl, pH 8.0 was added. The reaction mixture was then passed over a small column containing 1 ml of Protein G-sepharose 4B (Pharmacia Uppsala, Sweden) previously equilibrated with 200 mM Tris/HCl, pH 8.0. The flow through fraction was collected, dialysed against PBS, pH 6.8 and concentrated to 400 »l. This solution was further purified by HPLC on a TSK3000 (Pharmacia LKB Biotechnology AB, Uppsala, Sweden) column to give a single protein species of about 50000 Da molecular weight.

### Example 7

### Generation of F_{ab}-fragments by pepsin treatment

To 0.5 ml of a solution of the monoclonal antibody "htr-9" (10 mg/ml) [prepared as described in examples 1-4] in 100 mM sodiumacetate, pH 4.0, 100 »g of Pepsin (Merck, Darmstadt, BRD) in 100 »l 0.15M sodiumchloride were added. The mixture was incubated for 24 hours, at room temperature and then dialyzed against 200 mM Tris/HCl, pH 8.0 and passed over a protein G column as described in example 6. The flow through fraction (2 ml) was purged with nitrogen gas and incubated with 40 »l of a 1M solution of Dithiothreitol (Sigma) under nitrogen gas for 4 hours at room temperature. The mixture was then dialyzed against PBS pH 6.8, concentrated and purifid further by HPLC as described in example 6.

## Claims

1. A compound which is a monoclonal antibody or a fragment thereof which binds to a receptor for human Tumor Necrosis Factor (hTNF-R) obtainable by adsorption of a lysate of HL60 cells on an Affigel column carrying recombinant human TNF.

2. A compound according to claim 1 wherein the monoclonal antibody is of the IgM or the IgG subtype and the fragment is a fragment of said subtypes.

3. A compound according to claim 2 wherein the monoclonal antibody is of the IgM subtype and whose EC₅₀ of downmodulation on HL 60 cells [ATCC No. CCL 240] is about 30 ng/ml

4. A compound according to claim 2 wherein the monoclonal antibody is of the IgG subtype and whose EC₅₀ of downmodulation on HL 60 cells [ATCC No. CCL 240] is less than 1 »g/ml.

5. A compound according to claim 2 wherein the monoclonal antibody is of the IgM subtype and whose EC₅₀ of downmodulation on HEp-2 cells [ATCC No. CCL 23] is in the range of 30 ng/ml to 90 ng/ml.

6. A compound according to claim 2 wherein the monoclonal antibody is of the IgG subtype and whose EC₅₀ of downmodulation on HEp-2 cells [ATCC No. CCL 23] is about 1 »g/ml.

7. A compound according to one of claims 1-6 which does not cause downmodulation on murine cells.

8. A compound according to one of claims 1-7 which does not cause downmodulation on WEHI-164 [ATCC No. CRL 1751] cells.

9. A compound according to claims 1 to 8 coupled to a fluorescent dye, an enzyme or a radioactive substance.

10. Hybridoma cell lines secreting a monoclonal antibody according to one of claims 1-8.

11. A compound according to one of claims 1-9 for use as a therapeutically active substance or as a diagnostic tool.

12. A process for the preparation of a compound according to one of claims 1-8 which process comprises screening hybridoma cell lines with a radioimmuno-, enzymimmuno-, dotimmuno- or down modulation assay and determining an antibody as defined in claim 1 in the supernatant of such hybridoma cell lines as known in the art.

13. A pharmaceutical composition containing a compound according to one of claims 1-8 and a non-toxic, inert, therapeutically acceptable carrier material.

14. The use of a compound according to one of claims 1-8 in the preparation of a pharmaceutical composition for the treatment of septic shock.

15. The use of a compound according to one of claims 1-8 for the purification of the human TNF receptor.

## Patentansprüche

1. Eine Verbindung, die ein monoklonaler Antikörper oder ein Fragment davon ist, die an einen Rezeptor für menschlichen Tumornekrosefaktor (hTNF-R) bindet und durch Adsorption eines Lysates von HL 60 Zellen an eine Affigelsäule, die rekombinantes menschliches TNF trägt, erhältlich ist.

2. Eine Verbindung gemäss Anspruch 1, wobei der monoklonale Antikörper vom IgM oder IgG Subtyp ist und das Fragment ein Fragment besagten Subtyps ist.

3. Eine Verbindung gemäss Anspruch 2, wobei der monoklonale Antikörper vom IgM Subtyp ist und dessen EC₅₀ der Niederregelung an HL 60 Zellen [ATCC Nr. CCL 240] etwa 30 ng/ml ist.

4. Eine Verbindung gemäss Anspruch 2, wobei der monoklonale Antikörper vom IgG Subtyp ist und dessen EC₅₀ der Niederregelung an HL 60 Zellen [ATCC Nr. CCL 240] weniger als 1 »g/ml ist.

5. Eine Verbindung gemäss Anspruch 2, wobei der monoklonale Antikörper vom IgM Subtyp ist und dessen EC₅₀ der Niederregelung an HEp-2 Zellen [ATCC Nr. CCL 23] im Bereich von 30 ng/ml bis 90 ng/ml ist.

6. Eine Verbindung gemäss Anspruch 2, wobei der monoklonale Antikörper vom IgG Subtyp ist und dessen EC₅₀ der Niederregelung an HEp-2 Zellen [ATCC Nr. CCL 23] etwa 1 »g/ml ist.

7. Eine Verbindung gemäss einem der Ansprüche 1-6, die keine Niederregelung an Mäusezellen verursacht.

8. Eine Verbindung gemäss einem der Ansprüche 1-7, die keine Niederregelung an WEHI-164 [ATCC Nr. CRL 1751] Zellen verursacht.

9. Eine Verbindung gemäss den Ansprüchen 1 bis 8, die an einen fluoreszierenden Farbstoff, ein Enzym oder eine radioaktive Substanz gekoppelt ist.

10. Hybridomazelllinien, die einen Antikörper gemäss einem der Ansprüche 1 bis 8 sekretieren.

11. Eine Verbindung gemäss einem der Ansprüche 1-9 zur Verwendung als therapeutisch wirksame Substanz oder ein diagnostisches Mittel.

12. Ein Verfahren zur Herstellung einer Verbindung gemäss einem der Ansprüche 1-8, das das wie in der Technik bekannte Absuchen von Hybridomazelllinien mittels einem Radioimmun-, Enzymimmun-, Dotimmun- oder Niederregelungsnachweis und Bestimmen eines wie in Anspruch 1 definierten Antikörpers im Überstand solcher Hybridomzelllinien umfasst.

13. Eine pharmazeutische Zusammensetzung, die eine Verbindung gemäss einem der Ansprüche 1 bis 8 und einen nicht toxischen, inerten, therapeutisch verträglichen Träger enthält.

14. Die Verwendung einer Verbindung gemäss einem der Ansprüche 1 bis 8 bei der Herstellung einer pharmazeutischen Zusammensetzung zur Behandlung von septischem Schock.

15. Die Verwendung einer Verbindung gemäss einem der Ansprüche 1 bis 8 zur Reinigung von menschlichem TNF-Rezeptor.

## Revendications

1. Composé qui est un anticorps monoclonal ou un fragment de celui-ci, qui se fixe à un récepteur du facteur de nécrose tumorale humain (hTNF-R), pouvant être obtenu par adsorption d'un lysat de cellules HL60 sur une colonne d'Affigel portant du TNF humain recombinant.

2. Composé selon la revendication 1, caractérisé en ce que l'anticorps monoclonal appartient à la sous-classe des IgM ou des IgG et le fragment est un fragment appartenant à ces sous-classes.

3. Composé selon la revendication 2, caractérisé en ce que l'anticorps monoclonal appartient à la sous-classe des IgM et présente une CE₅₀ de répression sur des cellules HL60 (n° ATCC CCL 240) d'environ 30 ng/ml.

4. Composé selon la revendication 2, caractérisé en ce que l'anticorps monoclonal appartient à la sous-classe des IgG et présente une CE₅₀ de répression sur des cellules HL60 (n° ATCC CCL 240) inférieure à 1 »g/ml.

5. Composé selon la revendication 2, caractérisé en ce que l'anticorps monoclonal appartient à la sous-classe des IgM et présente une CE₅₀ de répression sur des cellules HEp-2 (n° ATCC CCL 23) dans la plage allant de 30 ng/ml à 90 ng/ml.

6. Composé selon la revendication 2, caractérisé en ce que l'anticorps monoclonal appartient à la sous-classe des IgG et présente une CE₅₀ de répression sur des cellules HEp-2 (n° ATCC CCL 23) d'environ 1 »g/ml.

7. Composé selon l'une des revendications 1 à 6, qui ne provoque pas de répression sur des cellules murines.

8. Composé selon l'une des revendications 1 à 7, qui ne provoque pas de répression sur des cellules WEHI-164 (n° ATCC CRL 1751).

9. Composé selon les revendications 1 à 8, couplé avec un colorant fluorescent, une enzyme ou une substance radioactive.

10. Lignées d'hybridomes sécrétant un anticorps monoclonal selon l'une des revendications 1 à 8.

11. Composé selon l'une des revendications 1 à 9, pour utilisation en tant que substance thérapeutiquement active ou en tant qu'outil diagnostique.

12. Procédé pour la préparation d'un composé selon l'une des revendications 1 à 8, lequel procédé comprend le criblage de lignées d'hybridomes à l'aide d'un essai radioimmunologique, immunoenzymatique, immunodot ou de répression, et la détermination d'un anticorps tel que défini dans la revendication 1, dans le surnageant de ces lignées d'hybridomes, comme il est connu dans la technique.

13. Composition pharmaceutique contenant un compose selon l'une des revendications 1 à 8 et un véhicule inerte, non toxique, thérapeutiquement acceptable.

14. Utilisation d'un composé selon l'une des revendications 1 à 8, dans la préparation d'une composition pharmaceutique pour le traitement du choc septique.

15. Utilisation d'un composé selon l'une des revendications 1 à 8, pour la purification du récepteur de TNF humain.
